# EUROPEAN PATENT SPECIFICATION

(11) **EP 1 002 531 B1**
(45) Date of publication and mention of the grant of the patent: **02.05.2003**
(21) Application number: 98308779.2
(22) Date of filing: 27.10.1998
(51) Int. Cl.: A61K 31/18, A61K 31/63, A61K 9/08, A61K 47/10, A61K 47/18, A61K 47/20, A61P 29/00

(54) **A water-miscible composition of nimesulide**
Wassermischbare Arzneizusammensetzung von Nimesulide
Composition miscible à l'eau de nimesulide

(43) Date of publication of application: 24.05.2000
(73) Proprietor: PANACEA BIOTEC LIMITED, New Dehli 110001 (IN)
(72) Inventor: Singh, Amarjit, New Delhi - 110001 (IN); JAIN, Rajesh, New Delhi - 110001 (IN)
(74) Representative: Grant, Anne Rosemary

(56) References cited:
- EP-A- 0 868 915
- EP-A- 0 869 117
- WO-A-95/34533
- WO-A-98/37868
- WO-A-98/37879
- US-A- 5 688 829
- PATENT ABSTRACTS OF JAPAN vol. 098, no. 014, 31 December 1998 & JP 10 245337 A (PANACEA BIOTEC LTD.,INDIA), 14 September 1998
- BAUER, FROEMMING, FUEHRER: "Lehrbuch der Pharmazeutischen Technologie", 1999, WISSENSCHAFTLICHE VERLAGSGESELLSCHAFT MBH STUTTGART, STUTTGART
- H. SUCKER, P. FUCHS, P. SPEISER: "Pharmazeutische Technologie", 1991, GEORG THIEME VERLAG STUTTGART, STUTTGART

## Description

The present invention relates to novel therapeutic non intensely coloured, water miscible injectable analgesic pharmaceutical compositions of the Non-steroidal anti-inflammatory drug Nimesulide and a process for the manufacture of such compositions. The analgesic injectable composition is very useful in mammals particularly in humans for the treatment of acute painful conditions like post operative trauma, pain associated with cancer, sports injuries, migraine headache, neurological pain, pain associated with sciatica and spondylitis. For these indications some modified route of administrations may also be construed.

Non-steroidal anti-inflammatory drugs such as those belonging to the category of Cyclo-oxygenase-2 inhibitors including Nimesulide are highly hydrophobic compounds and readily precipitate even in the presence of minor amounts of water.

It is therefore very difficult to formulate Non-steroidal anti-inflammatory drugs which are inhibitors of Cyclo-oxygenase-2 as an injection for intramuscular or intravenous use.

In the past, efforts have been made to make an injectable composition of Nimesulide.

An injectable formulation of Nimesulide has been reported in the prior art PCT Patent No. WO 95/34533 which utilizes a salt form of Nimesulide with L-lysine which is in turn further complexed with cyclodextrins which may be dissolved in water to give an injectable preparation. The maximum solubility achieved by this injectable composition was reported to be 2.4 mg/ml which is not sufficient for intramuscular administration as it would require very large volumes to administer therapeutic doses. Moreover making a salt form of Nimesulide and then combining with Cyclodextrins not only makes the process cumbersome but also increases the cost of the formulations.

Another reference ( Daffonchio.L et al. Inflammatory Research 45 : 259-264; 1995) wherein Nimesulide is dissolved in saline for intravenous administration for experimental studies in animals also describes only very dilute solutions which cannot deliver therapeutic doses in humans.

The disadvantages of WO 95/34533 and problems associated therewith were overcome by the invention described in US Patent No. 5,688,829 ( Corresponding Korean Application No. 96-48013, Australian Application No. 67993/96 and Japanese Patent Application No. 8-290585, all pending) by the inventors of the present invention.

This invention utilizing solubilization techniques was able to achieve sufficiently high concentrations of Nimesulide suitable to deliver therapeutic doses in conveniently small volumes using a base which was oily in nature without using water and without any salt form or complexing agents of Nimesulide.

The present invention comprises a composition wherein all the ingredients of the base are hydrophilic. The hydrophilic base serves the advantage of better miscibility with body fluids, faster drug disposition and better compatibility with the tissue environment.

It is an objective of the present invention to provide an injectable analgesic hydrophilic composition of Non-steroidal anti-inflammatory drugs which are non-intensely coloured having better miscibility with body fluids. Such hydrophilic compositions described herein can be administered by intravenous routes also in addition to intramuscular route.

It is another objective of the present invention to provide a novel process for the preparation of a parenteral hydrophilic injectable composition of NSAIDs.

The invention comprises a novel injectable aqueous miscible composition of Nimesulide which comprises:
a) nimesulide;
b) one or more alkyl amides, alkyl sulphoxides or pyrrolidones;
c) one or more glycols selected from the group comprising polyethylene glycol MW 200 to 6000, propylene glycol, hexylene glycol, butylene glycol and polyethylene glycol 660 hydroxy stearate;
d) 0 to 20% water
suitable for intramuscular and intravenous use.

The Alkyl amides, Alkyl sulphoxides or pyrrolidones in accordance with the present invention are preferably selected from the group comprising Dimethylacetamide Dimethylformamide and Dimethylsulphoxide or N-Methyl Pyrrolidone.

The glycols in accordance with the present invention are preferably selected from the group Polyethylene Glycol MW 200 to 6000, Propylene Glycol, Hexylene glycols, Butylene glycols and Glycol derivatives such as Polyethylene Glycol 660 hydroxy stearate (commercially available as Solutrol HS15).

Beside the composition may also comprise one or more of the following conventional additional ingredients - Surfactants, hydrophilic polymers, solubility enhancing agents i.e. Glycerine, various grades of Polyethylene oxides, β-cyclodextrins like sulfo butyl ether-β-cyclodextrin, Transcutol and Glycofurol, tonicity adjusting agents, local anesthetics, pH adjusting agents and buffers.

Preferably Nimesulide is present in the composition from 0.1 to 10% w/v.

More preferably Nimesulide is present from 0.5 to 8% w/v.

More preferably Nimesulide is present in the composition from 1.2 to 4.8% w/v. Preferably the composition in accordance with the present invention comprises Alkyl amides/Alkyl sulphoxides or pyrrolidones from 2.0% to 95% w/v.

More preferably the composition comprises Alkyl amides, Alkyl sulphoxides or pyrrolidones from 5% to 90%.

More preferably Alkyl amides, Alkyl sulphoxides or pyrrolidones are present from 10% to 20% w/v.

Preferably Glycols are present in the composition from 0.1% to 95% w/v.

In a preferred embodiment of the invention there is described an injectable water miscible non-intensely coloured analgesic pharmaceutical composition of Nimesulide which comprises :

| | |
|---|---|
| Nimesulide from | 0.1 to 10% w/v. |
| Dimethylacetamide from | 2.0 to 90% w/v. |
| Polyethylene Glycols from | 0.1 to 95% w/v. |
| Water from | 0 to 20% w/v. |

In a preferred embodiment, the drug is dissolved in an oily phase and emulsified in aqueous phase using surfactants including lecithins leading to microemulsion or emulsion suitable for intravenous use.

In accordance with the present invention there is also described a novel process for the manufacture of an injectable water miscible analgesic pharmaceutical composition of Nimesulide.

The process comprises:
a) Dissolving 0.1 to 10% w/v of Nimesulide in 2 to 90 % of one or more Alkyl amide, Alkylsulphoxide or pyrrolidones and adding thereto freshly distilled 0 to 4 % Benzyl Alcohol while stirring.
b) Adding to the resultant solution 0.1 to 60% of one or more glycols and mixing.
c) Adding to the solution Hydrochloric Acid solution slowly and adding thereto one or more Glycols to make volume up to 95% of the batch.
d) Adding to the resultant solution Sodium hydroxide/Hydrochloric Acid to adjust pH to a range between 1.5 to 5.5 and adding one or more Glycols to make up the final volume.

Preferably, the pH is adjusted to from 2.0 to 3.0.

Preferably, the process is carried out at a temperature of from 10 to 60°C and at atmospheric pressure.

Further the resultant solution may be filtered through 0.45 micron nylon membrane filter using a 6 micron glass fibre pre-filter. Collect the filtered solution in a clean fibre-free vessel. The solution may then be filled in fibre-free sterile 2 or 3 ml amber USP Type 1 glass ampoules with pre and post filling nitrogen flushing.

Sterilise the ampoules by autoclaving. Optically inspect all the ampoules and after release by Quality Control Deptt. label the good ones.

The invention will now be described by the following examples for injectable analgesic composition of Nimesulide.

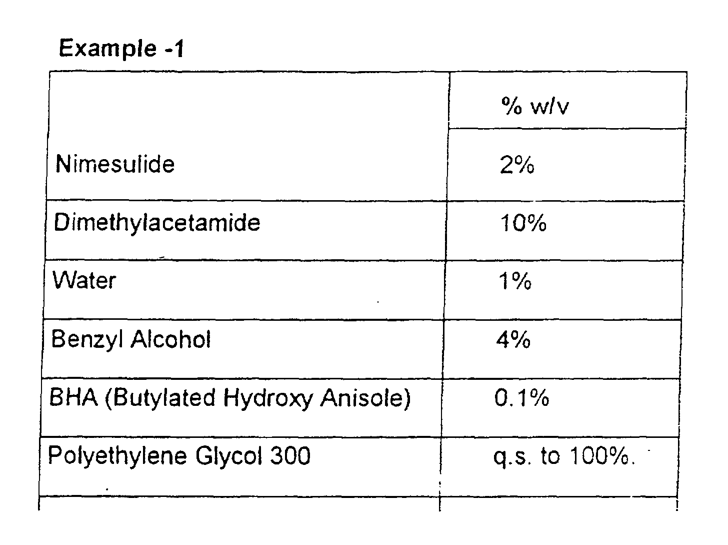

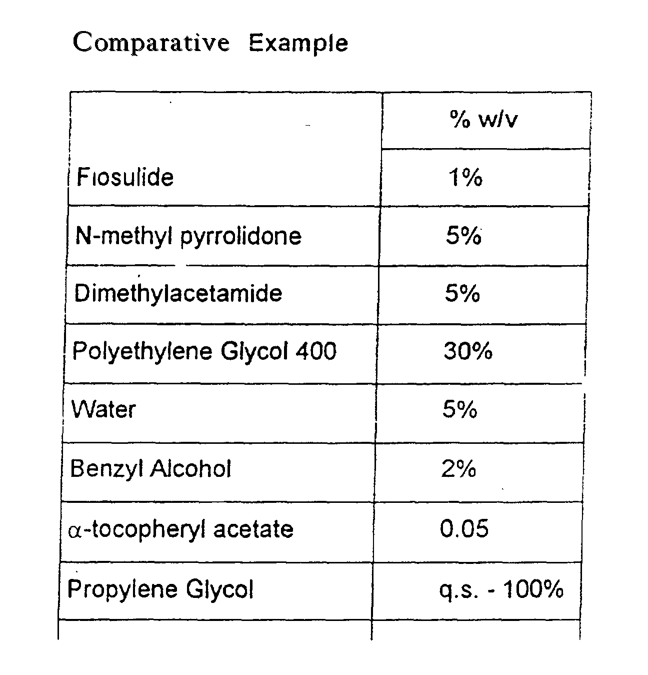

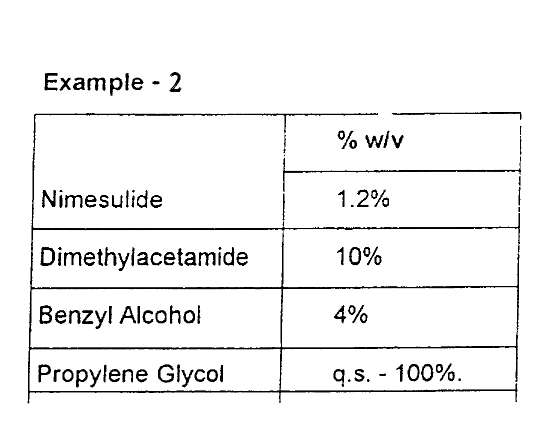

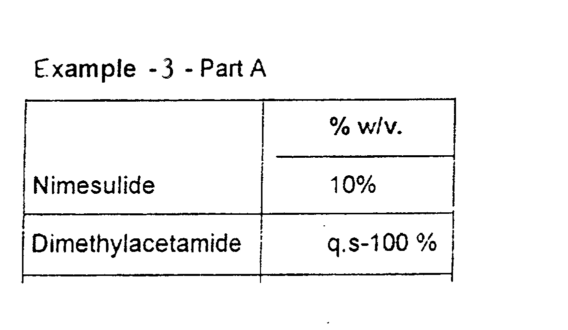

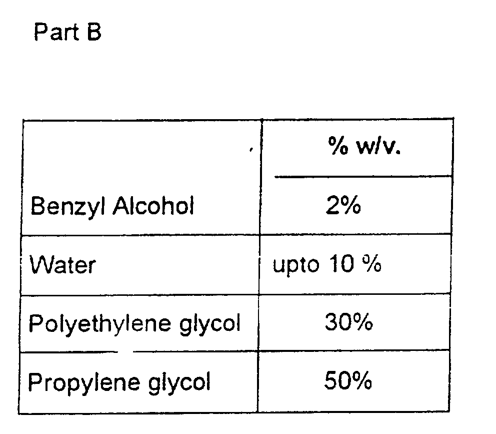

This composition is in the form of a kit to be the ingredients of which are to be mixed prior to administration.

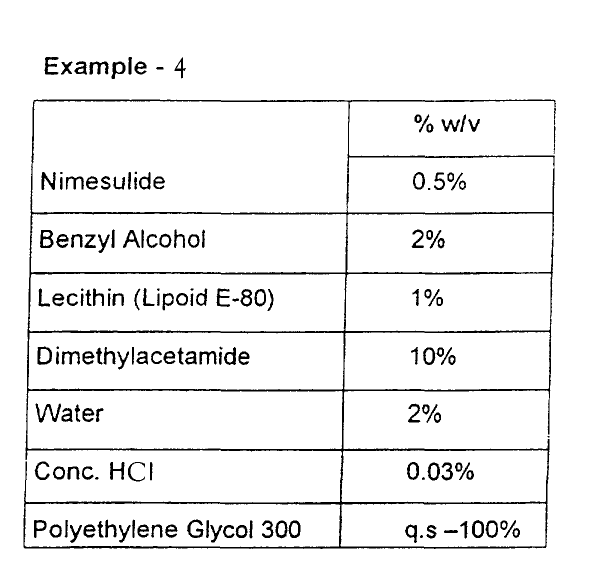

On affecting Acute Toxicity studies on Balb/C Mice by intra peritoneal route the LD₅₀ was found to be 160 mg/kg, ED₅₀ = 3 mg/kg with therapeutic index = 53.3 in mice. This demonstrates high safety of the present invention. The injectable analgesic composition, according to the present invention, on preliminary animals and preclinical trails as shown to posses marked analgesic activity. Further it has been found to non-toxic even on repeated applications on same site.

No incidence of tissue necrosis or any other side effect was observed. The analgesic dose range from 0.1 mg/kg to 8.4 mg/kg.

## Claims

1. A parenteral water-miscible injectable composition of non-steroidal anti-inflammatory drugs belonging to cyclo-oxygenase-2 inhibitor category which comprises:
a) nimesulide;
b) one or more alkyl amides, alkyl sulphoxides or pyrrolidones;
c) one or more glycols selected from the group comprising polyethylene glycol MW 200 to 6000, propylene glycol, hexylene glycol, butylene glycol and polyethylene glycol 660 hydroxy stearate;
d) 0 to 20% water
suitable for intramuscular and intravenous use.

2. An injectable composition as claimed in claim 1 wherein nimesulide is present in the composition from 0.1 to 10% w/v.

3. An injectable composition as claimed in any one of the preceding claims wherein said alkyl sulphoxides, alkyl amides or pyrrolidones are selected from the group comprising dimethylacetamide, dimethylformamide, dimethylsulphoxide and N-methyl pyrrolidone.

4. An injectable composition as claimed in any one of the preceding claims wherein said alkyl amide is dimethylacetamide.

5. An injectable composition as claimed in any one of the preceding claims wherein said alkyl sulphoxide, alkyl amide or pyrrolidone is present in the composition from 2 to 90%.

6. An injectable composition as claimed in any one of the preceding claims wherein said glycol is polyethylene glycol 300.

7. An injectable composition as claimed in any one of the preceding claims wherein said glycols are present in the composition from 0.1 to 95%.

8. A parenteral water miscible injectable composition according to claim 1 comprising:
| | |
|---|---|
| nimesulide | 0.1 to 10% w/v |
| dimethylacetamide | 2.0 to 90% w/v |
| polyethylene glycol 300 | 0.1 to 95% w/v |
| water | 0 to 20% w/v. |

9. A composition as claimed in claim 8 comprising 0 to 4% benzyl alcohol.

10. A process for the manufacture of a parenteral water-miscible injectable composition of nimesulide comprising:
a) dissolving 0.1 to 10% w/v of a nimesulide in 2 to 90% of one or more alkyl amides, alkylsulphoxides or pyrrolidones and adding thereto freshly distilled 0 to 4% benzyl alcohol while stirring;
b) adding to the resulting solution 0.1 to 60% of one or more glycols selected from the group comprising polyethylene glycol MW 200 to 6000, propylene glycol, hexylene glycol, butylene glycol and polyethylene glycol 660 hydroxy stearate and mixing;
c) adding to the solution hydrochloric acid solution slowly and adding thereto one or more glycols selected from the group comprising polyethylene glycol MW 200 to 6000, propylene glycol, hexylene glycol, butylene glycol and polyethylene glycol 660 hydroxy stearate to make volume up to 95% of the batch;
d) adding to the resulting solution sodium hydroxide/hydrochloric acid to adjust pH to a range between 1.5 to 5.5 and adding one or more glycols selected from the group comprising polyethylene glycol MW 200 to 6000, propylene glycol, hexylene glycol, butylene glycol and polyethylene glycol 660 hydroxy stearate; to make up the final volume.

11. A process as claimed in claim 10 which comprises filtering the resultant solution in step d).

12. A process as claimed in claim 11 comprising filling the filtered solution in suitable containers followed by subjecting such containers to terminal sterilization.

13. A process as claimed in claim 10 wherein the process is carried out at a temperature of from 10 to 60°C and at atmospheric pressure.

14. A kit of parts comprising:
(i) nimesulide 10%, dimethylacetamide (to 100%); and
(ii) benzylalcohol 2%, water up to 10%, polyethylene glycol 30% and propylene glycol 50%
in a form such that the components are mixed prior to administration.

15. A composition used for the process for treating a non-steroidal anti inflammatory drug indicated condition or symptom with less or substantially no side effects and at a lower dosage through intravenous route said process comprising administering an effective amount of a parenteral water-miscible injectable composition of a non-steroidal anti-inflammatory drugs belong to cyclo-oxygenase-2 inhibitor category which comprises:
a) nimesulide;
b) one or more alkyl amides, alkyl sulphoxides or pyrrolidones;
c) one or more glycols selected from the group comprising polyethylene glycol MW 200 to 6000, propylene glycol, hexylene glycol, butylene glycol and polyethylene glycol 660 hydroxy stearate;
d) 0 to 20% of water.

16. Use of
a) nimesulide;
b) one or more alkyl amides, alkyl sulphoxides or pyrrolidones;
c) one or more glycols selected from the group comprising polyethylene glycol MW 200 to 6000, propylene glycol, hexylene glycol, butylene glycol and polyethylene glycol 660 hydroxy stearate;
d) 0 to 20% of water
in the manufacture of an analgesic composition for treating a non-steroidal anti-inflammatory drug indicated condition or symptom with less or substantially no side effects and at a lower dosage through intravenous route.

## Patentansprüche

1. Parenterale wassermischbare injizierbare Zusammensetzung nichtsteroidaler entzündungshemmender Arzneimittel der Klasse der Cyclooxygenase-2-Inhibitoren, umfassend:
a) Nimesulid;
b) wenigstens ein Alkylamid, Alkylsulfoxid oder Pyrrolidon;
c) wenigstens ein unter Polyethylenglycol MW 200 bis 6000, Propylenglycol, Hexylenglycol, Butylenglycol und Polyethylenglycol 660-hydroxystearat ausgewähltes Glycol;
d) 0 bis 20 % Wasser,
die geeignet ist zur intramuskulären und intravenösen Anwendung.

2. Injizierbare Zusammensetzung nach Anspruch 1, wobei das Nimesulid in der Zusammensetzung in einer Menge von 0,1 bis 10 % Gew./Vol. vorliegt.

3. Injizierbare Zusammensetzung nach einem der vorhergehenden Ansprüche, wobei das Alkylsulfoxid, Alkylamid oder Pyrrolidon unter Dimethylacetamid, Dimethylformamid, Dimethylsulfoxid und N-Methylpyrrolidon ausgewählt ist.

4. Injizierbare Zusammensetzung nach einem der vorhergehenden Ansprüche, wobei das Alkylamid Dimethylacetamid ist.

5. Injizierbare Zusammensetzung nach einem der vorhergehenden Ansprüche, wobei das Alkylsulfoxid, Alkylamid oder Pyrrolidon in der Zusammensetzung in einer Menge von 2 bis 90 % vorliegt.

6. Injizierbare Zusammensetzung nach einem der vorhergehenden Ansprüche, wobei das Glycol Polyethylenglycol 300 ist.

7. Injizierbare Zusammensetzung nach einem der vorhergehenden Ansprüche, wobei das Glycol in der Zusammensetzung in einer Menge von 0,1 bis 95 % vorliegt.

8. Parenterale wassermischbare injizierbare Zusammensetzung nach Anspruch 1, umfassend:
| | |
|---|---|
| Nimesulid: | 0,1 bis 10 % Gew./Vol. |
| Dimethylacetamid: | 2,0 bis 90 % Gew./Vol. |
| Polyethylenglycol 300 | 0,1 bis 95 % Gew./Vol. |
| Wasser | 0 bis 20 % Gew./Vol. |

9. Zusammensetzung nach Anspruch 8, umfassend 0 bis 4 % Benzylalkohol.

10. Verfahren zur Herstellung einer parenteralen wassermischbaren injizierbaren Zusammensetzung von Nimesulid, bei dem man
a) 0,1 bis 10 % Gew./Vol. Nimesulid in 2 bis 90 % wenigstens eines Alkylamids, Alkylsulfoxids oder Pyrrolidons auflöst und unter Rühren mit 0 bis 4 % frisch destilliertem Benzylalkohol versetzt;
b) die erhaltene Lösung mit 0,1 bis 60 % wenigstens eines unter Polyethylenglycol MW 200 bis 6000, Propylenglycol, Hexylenglycol, Butylenglycol und Polyethylenglycol 660-hydroxystearat ausgewählten Glycols versetzt und mischt;
c) die Lösung langsam mit Salzsäure versetzt und darauf mit wenigstens einem unter Polyethylenglycol MW 200 bis 6000, Propylenglycol, Hexylenglycol, Butylenglycol und Polyethylenglycol 660-hydroxystearat ausgewählten Glycol versetzt, um das Volumen auf 95 % der Ansatzgröße aufzufüllen;
d) die erhaltene Lösung mit Natriumhydroxid/Salzsäure versetzt, um den pH auf einen Wert zwischen 1,5 bis 5,5 einzustellen, und mit wenigstens einem unter Polyethylenglycol MW 200 bis 6000, Propylenglycol, Hexylenglycol, Butylenglycol und Polyethylenglycol 660-hydroxystearat ausgewählten Glycol versetzt, um das endgültige Volumen einzustellen.

11. Verfahren nach Anspruch 10, bei dem man die im Schritt d) erhaltene Lösung filtriert.

12. Verfahren nach Anspruch 11, bei dem man die filtrierte Lösung in geeignete Behälter füllt und diese Behälter dann einer abschließenden Sterilisation unterzieht.

13. Verfahren nach Anspruch 10, wobei man das Verfahren bei einer Temperatur von 10 bis 60 °C und bei Atmosphärendruck durchführt.

14. Kit mit mehreren Teilen, umfassend
(i) Nimesulid 10 %, Dimethylsulfoxid (auf 100 %); und
(ii) Benzylalkohol 2 %, Wasser auf 10 %, Polyethylenglycol 30 % und Propylenglycol 50 %
in geeigneter Form, um die Komponenten vor der Verabreichung zu mischen.

15. Zusammensetzung zur Verwendung in einem Verfahren zur Behandlung eines Krankheitsbildes oder Symtoms, bei dem ein nichtsteroidales entzündungshemmendes Arzneimittel angezeigt ist, mit weniger oder im Wesentlichen ohne Nebenwirkungen und bei geringerer Dosis durch intravenöse Verabreichung, bei dem man eine wirksame Menge einer parenteralen wassermischbaren injizierbaren Zusammensetzung eines nichtsteroidalen entzündungshemmenden Arzneimittels der Klasse der Cyclooxygenase-2-lnhibitoren verabreicht, die umfasst:
a) Nimesulid;
b) wenigstens ein Alkylamid, Alkylsulfoxid oder Pyrrolidon;
c) wenigstens ein unter Polyethylenglycolen MW 200 bis 6000, Propylenglycol, Hexylenglycol, Butylenglycol und Polyethylenglycol 660-hydroxystearat ausgewähltes Glycol;
d) 0 bis 20 % Wasser.

16. Verwendung von
a) Nimesulid;
b) wenigstens einem Alkylamid, Alkylsulfoxid oder Pyrrolidon;
c) wenigstens einem unter Polyethylenglycolen MW 200 bis 6000, Propylenglycol, Hexylenglycol, Butylenglycol und Polyethylenglycol 660-hydroxystearat ausgewählten Glycol;
d) 0 bis 20 % Wasser
zur Herstellung einer analgetischen Zusammensetzung zur Behandlung eines Krankheitsbildes oder Symtoms, bei dem ein nichtsteroidales entzündungshemmendes Arzneimittel angezeigt ist, mit weniger oder im Wesentlichen ohne Nebenwirkungen und bei geringerer Dosis durch intravenöse Verabreichung.

## Revendications

1. Composition injectable par voie parentérale et miscible dans l'eau de médicaments anti-inflammatoires non stéroïdiens appartenant à la catégorie des inhibiteurs de la cyclo-oxygénase 2 qui comprend :
a) le nimésulide ;
b) un ou plusieurs alkylamides, alkylsulfoxydes ou pyrrolidones ;
c) un ou plusieurs glycols choisis dans le groupe comprenant le polyéthylène glycol (masse moléculaire de 200 à 6000), le propylène glycol, l'hexylène glycol, le butylène glycol et l'hydroxystéarate de polyéthylène glycol 660 ;
d) de 0 à 20 % d'eau
adaptée pour un usage par voie intramusculaire et intraveineuse.

2. Composition injectable selon la revendication 1, dans laquelle le nimésulide est présent dans la composition dans une quantité allant de 0,1 à 10 % p/v;

3. Composition injectable selon l'une quelconque des revendications précédentes, dans laquelle lesdits alkylsulfoxydes, alkylamides ou lesdites pyrrolidones sont choisis dans le groupe comprenant le diméthylacétamide, le diméthylformamide, le diméthylsulfoxyde et la N-méthylpyrrolidone.

4. Composition injectable selon l'une quelconque des revendications précédentes, dans laquelle ledit alkylamide est le diméthylacétamide.

5. Composition injectable selon l'une quelconque des revendications précédentes, dans laquelle ledit alkylsulfoxyde, alkylamide ou ladite pyrrolidone est présent(e) dans la composition dans une quantité allant de 2 à 90 %.

6. Composition injectable selon l'une quelconque des revendications précédentes, dans laquelle ledit glycol est le polyéthylène glycol 300.

7. Composition injectable selon l'une quelconque des revendications précédentes, dans laquelle lesdits glycols sont présents dans la composition dans une quantité allant de 0,1 à 95 %.

8. Composition injectable par voie parentérale et miscible dans l'eau selon la revendication 1, comprenant :
| | |
|---|---|
| Nimésulide | 0,1 à 10 % p/v |
| Diméthylacétamide | 2,0 à 90 % p/v |
| Polyéthylène glycol 300 | 0,1 à 95 % p/v |
| Eau | 0 à 20 % p/v |

9. Composition selon la revendication 8, comprenant de 0 à 4 % d'alcool benzylique.

10. Procédé pour la fabrication d'une composition injectable par voie parentérale et miscible dans l'eau de nimésulide comprenant les étapes consistant à :
a) dissoudre de 0,1 à 10 % p/v d'un nimésulide dans 2 à 90 % d'un ou de plusieurs alkylamides, alkylsulfoxydes ou pyrrolidones et ajouter à ceux-ci de 0 à 4 % d'alcool benzylique fraîchement distillé sous agitation ;
b) ajouter à la solution obtenue de 0,1 à 60 % d'un ou de plusieurs glycols choisis dans le groupe comprenant le polyéthylène glycol (masse moléculaire de 200 à 6000), le propylène glycol, l'hexylène glycol, le butylène glycol et l'hydroxystéarate de polyéthylène glycol 660 et les mélanger ;
c) ajouter lentement à la solution une solution d'acide chlorhydrique et y ajouter un ou plusieurs glycols choisis dans le groupe comprenant le polyéthylène glycol (masse moléculaire de 200 à 6000), le propylène glycol, l'hexylène glycol, le butylène glycol et l'hydroxystéarate de polyéthylène glycol 660 pour augmenter le volume jusqu'à 95 % du lot ;
d) ajouter à la solution obtenue un mélange d'hydroxyde de sodiumlacide chlorhydrique pour régler le pH dans une plage comprise entre 1,5 et 5,5 et ajouter un ou plusieurs glycols choisis dans le groupe comprenant le polyéthylène glycol (masse moléculaire de 200 à 6000), le propylène glycol, l'hexylène glycol, le butylène glycol et l'hydroxystéarate de polyéthylène glycol 660 pour établir le volume final.

11. Procédé selon la revendication 10, qui comprend le filtrage de la solution obtenue à l'étape d).

12. Procédé selon la revendication 11, comprenant le remplissage de récipients adaptés avec la solution filtrée suivi de l'action consistant à soumettre ces récipients à une stérilisation finale.

13. Procédé selon la revendication 10, dans lequel le procédé est réalisé à une température allant de 10 à 60°C et sous pression atmosphérique.

14. Trousse de composants comprenant :
(i) du nimésulide à 10 %, du diméthylacétamide (jusqu'à 100 %) ; et
(ii) de l'alcool benzylique à 2 %, de l'eau jusqu'à 10 %, du polyéthylène glycol à 30 % et du propylène glycol à 50 %
sous une forme telle que les composants soient mélangés avant administration.

15. Composition utilisée pour le procédé destiné à traiter un état ou un symptôme pour lequel est indiqué un médicament anti-inflammatoire non stéroïdien avec moins d'effets secondaires ou sensiblement aucun effet secondaire et avec un dosage plus faible par voie intraveineuse, ledit procédé comprenant l'administration d'une quantité efficace d'une composition injectable par voie parentérale et miscible dans l'eau d'un médicament anti-inflammatoire non stéroïdien appartenant à la catégorie des inhibiteurs de la cyclo-oxygénase 2 qui comprend :
a) le nimésulide ;
b) un ou plusieurs alkylamides, alkysulfoxydes ou pyrrolidones ;
c) un ou plusieurs glycols choisis dans le groupe comprenant le polyéthylène glycol (masse moléculaire de 200 à 6000), le propylène glycol, l'hexylène glycol, le butylène glycol et l'hydroxystéarate de polyéthylène glycol 660 ;
d) de 0 à 20 % d'eau.

16. Utilisation de :
a) nimésulide ;
b) un ou plusieurs alkylamides, alkylsulfoxydes ou pyrrolidones ;
c) un ou plusieurs glycols choisis dans le groupe comprenant le polyéthylène glycol (masse moléculaire de 200 à 6000), le propylène glycol, l'hexylène glycol, le butylène glycol et l'hydroxystéarate de polyéthylène glycol 660 ;
4) de 0 à 20 % d'eau
dans la préparation d'une composition analgésique destinée au traitement d'un état ou un symptôme pour lequel est indiqué un médicament anti-inflammatoire non stéroïdien avec moins d'effets secondaires ou sensiblement aucun effet secondaire et à un dosage plus faible par voie intraveineuse.
